# EUROPEAN PATENT APPLICATION

(11) **EP 4 621 058 A1**
(43) Date of publication of application: **24.09.2025**
(21) Application number: 23891933.6
(22) Date of filing: 13.11.2023
(51) Int. Cl.: C12N 15/74, C07K 14/195, A61K 38/16, A61K 39/39, A61K 39/112, A61K 45/06, A61P 35/00, A61P 35/04

(54) **IMMUNOENHANCING SALMONELLA STRAIN FOR TREATMENT OF CANCER AND USE THEREOF**

(30) Priority: 14.11.2022 KR 20220151641
(71) Applicant: CNCure Co., Ltd., Jeollanam-do 58128 (KR)
(72) Inventor: MIN, Jung-Joon, Gwangju 61461 (KR); HONG, Yeongjin, Gwangju 61682 (KR); RHEE, Joon Haeng, Gwangju 61903 (KR); YOU, Sung-Hwan, Gwangju 61419 (KR)
(74) Representative: Müller, Christian Stefan Gerd
(86) International application number: PCT/KR2023/018120
(87) International publication number: WO 2024/106866

(57) **Abstract**

The present invention relates to a DNA construct including: a gene encoding a flagellin protein; and a gene encoding an immunoenhancer (adjuvant) protein. For effective cancer therapy by selectively killing only cancer cells, an attenuated Salmonella strain according to the present disclosure is designed to produce immunogenic substances in cancer tissue to induce a strong anti-cancer immune response, whereby tumor sizes in metastatic cancers as well as primary cancers can be significantly inhibited. Thus, the strain can be advantageously used in a prophylactic or therapeutic composition for improving survival rates.

## Description

### [Technical Field]

The present invention relates to an immunoenhancing salmonella strain for treatment of cancer and use thereof.

### [Background Art]

To date, most cancers have been treated through individual approaches such as surgical removal, radiation therapy, or chemotherapy, or by combinations thereof. Surgical procedures that remove most of the tumor can be highly effective for cancers located in specific areas such as the breast, colon, or skin. However, surgery is difficult to apply to tumors located in certain regions, such as the spine. Additionally, systemic chemotherapy, which is commonly used for cancers such as breast cancer, lung cancer, and testicular cancer, may cause side effects due to disruption of normal cell replication or metabolic processes. Furthermore, patients may develop resistance to the chemotherapeutic agents used in treatment.

Meanwhile, when cancer occurs in an organism, rapid angiogenesis and cellular growth within the body create environments where tumor tissue often suffers from incomplete vascularization and hypoxia. Such conditions are highly suitable for the proliferation of facultative anaerobic bacteria such as *Salmonella* spp. or *Escherichia coli.* As a result, current cancer therapies using tumor-targeting bacteria like *Salmonella* or *Clostridium* spp. rely on their ability to selectively proliferate within solid tumors. When tumor-lytic proteins or reporter proteins are introduced into such bacteria and the transformed strains are administered to an organism, it becomes possible to specifically target tumor tissues, detect them, and minimize toxicity to normal cells, thereby enhancing the therapeutic efficacy.

In nature, various bacterial pathogens secrete toxins that can cause disease in humans. Among these, *Salmonella enterica,* a bacterium closely associated with human dietary habits and known to inhabit the gastrointestinal tract of primates, including humans, is known to secrete an exotoxin called cytolysin. Cytolysin, a cytotoxic protein with a molecular weight of approximately 34 kDa, exhibits hemolytic activity by destroying red blood cells in the gastrointestinal tract and forms pores in the membranes of normal cells, leading to cell lysis. This can cause severe vascular inflammation and local tissue necrosis, potentially resulting in death. However, recent studies have shown that cytolysin isolated and purified from *Salmonella enterica* can selectively react with cancer tissues present in the gastrointestinal tract, inducing cancer cell death, and has gained attention as a promising next-generation anticancer agent. Therefore, genetically engineered bacteria that secrete the cytotoxic protein cytolysin have high potential as tumor-targeting anticancer therapies. Despite the potential for bacterial use in cancer diagnosis and therapy, research on expression vectors that allow diagnostic or therapeutic proteins to be selectively expressed in tumor tissues remains limited.

Accordingly, the present invention was devised to develop an effective cancer prevention or treatment by selectively targeting and killing cancer cells. It demonstrates that an attenuated *Salmonella* strain, engineered to produce immunostimulatory molecules within tumor tissues, can induce a strong antitumor immune response, thereby effectively treating both primary and metastatic cancers. The pharmaceutical composition of the present invention enhances survival by specifically targeting and killing tumors within the body, and is therefore expected to be widely used in the field of cancer treatment.

### [Disclosure]

### [Technical Problem]

The inventors of the present invention have made dedicated efforts to develop an effective cancer preventive or therapeutic agent by selectively killing only cancer cells. As a result, they confirmed that an attenuated *Salmonella* strain, engineered to produce immunostimulatory substances within tumor tissues, can induce a strong antitumor immune response and effectively treat not only primary tumors but also metastatic cancers. Through this discovery, they verified the ability to specifically target and eliminate cancer cells within a living system, thereby improving survival rates, which ultimately led to the completion of the present invention.

Accordingly, an object of the present invention is to provide a DNA construct comprising: a gene encoding a flagellin protein; and a gene encoding an adjuvant protein.

Other objects and advantages of the present invention will become more apparent from the following detailed description of the invention, the claims, and the accompanying drawings.

However, objects to be achieved by the present disclosure are not limited to the objects mentioned above, and other objects not mentioned above may be clearly understood by those skilled in the art from the following description.

### [Technical Solution]

Hereinafter, various embodiments described herein will be described with reference to figures. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present disclosure pertains.

Throughout the present specification, when a part is described as "comprising" a certain component, it is to be understood-unless expressly stated otherwise-that the term does not exclude the presence of other components, but rather allows for the inclusion of additional components.

According to one aspect of the present invention, the invention provides a DNA construct.

The DNA construct of the present invention comprises: a gene encoding a flagellin protein; and a gene encoding an adjuvant protein.

The inventors have conducted extensive research to develop an effective cancer preventive or therapeutic agent that selectively kills only cancer cells. As a result, they confirmed that an attenuated *Salmonella* strain, engineered to produce immunostimulatory substances that act selectively on cancer, can induce a strong antitumor immune response, effectively treating not only primary tumors but also metastatic cancers. Through this discovery of a novel and efficient method and composition for treating cancer within a living system, the present invention was completed.

According to the present invention, the flagellin and adjuvant may be expressed as a fusion protein using an expression vector, although the invention is not limited thereto.

In this specification, the term "fusion protein" refers to an artificially recombined protein that is expressed by linking one or more genes of other proteins to a protein. Fusion proteins are expected to exhibit synergistic effects by combining the functions of two or more proteins.

According to the present invention, in order to allow the fusion protein to be secreted from the bacterial strain, a signal peptide was added to the 5'-end of the fusion protein. Specifically, the signal peptide may be selected from the group consisting of: pectate lyase B (PelB), outer-membrane protein A (OmpA), heat-stable enterotoxin 2 (StII), endoxylanase, alkaline phosphatase (PhoA), outer-membrane protein F (OmpF), and outer-membrane pore protein E (PhoE).

In this specification, the term *"Salmonella"* refers to a genus of proteobacteria belonging to the family Enterobacteriaceae. These rod-shaped bacteria, measuring approximately 0.7 to 1.5 µm in diameter and 2 to 5 µm in length, primarily inhabit the digestive tracts of humans and animals.

As used in the present invention, "flagellin" refers, without limitation, to the globular protein that constitutes the helical filament of bacterial flagella. The molecular weight of flagellin varies greatly depending on the bacterial species (ranging from 30,000 to 70,000 Da), and in terms of amino acid composition, cysteine and tryptophan are generally absent. In the case of *Salmonella,* approximately half of the lysine residues are methylated. Flagellin exists in types such as flagellin A and B, and although it has various functions, it is known to possess immunostimulatory properties.

In the present invention, the flagellin is either flagellin A (FlaA) or flagellin B (FlaB).

In this specification, the term "cancer" refers to a condition characterized by uncontrolled cell growth, where such abnormal proliferation leads to the formation of a mass of cells known as a tumor. These tumors can invade surrounding tissues and, in severe cases, metastasize to other organs in the body. Scientifically, cancer may also be referred to as a neoplasm. Despite treatment through surgery, radiation therapy, or chemotherapy, cancer often remains incurable in many cases, causing significant suffering to patients and ultimately leading to death. Cancer is considered an intractable chronic disease. Its causes can be broadly divided into internal and external factors. While the exact mechanisms by which normal cells transform into cancer cells have not been fully elucidated, a significant proportion of cancers are known to arise due to environmental influences and other external factors. Internal factors include genetic predisposition and immunological conditions, while external factors encompass exposure to chemicals, radiation, and viruses. Genes associated with cancer development include oncogenes and tumor suppressor genes. Cancer arises when the balance between these two types of genes is disrupted due to the aforementioned internal or external influences. In the present invention, the targets for prevention, improvement, or treatment may include, but are not limited to: melanoma, fallopian tube cancer, brain cancer, small intestine cancer, esophageal cancer, lymphoma, gallbladder cancer, hematologic cancer, thyroid cancer, endocrine gland cancer, oral cancer, liver cancer, bile duct cancer, colon cancer, rectal cancer, cervical cancer, ovarian cancer, kidney cancer, gastric cancer, duodenal cancer, prostate cancer, breast cancer, brain tumors, lung cancer, anaplastic thyroid cancer, uterine cancer, colorectal cancer, bladder cancer, ureteral cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myeloid leukemia, and solitary plasmacytoma.

According to the present invention, the first promoter and the second promoter can be simultaneously induced by a single regulatory protein expressed under the control of separate promoters. As a result, compared to a case where the gene encoding the regulatory protein is operably linked downstream of the second promoter, the expression levels of proteins encoded by genes operably linked downstream of the first and second promoters in the host cell, or within the host cell, can be balanced. In this way, using the DNA construct of the present invention enables simultaneous diagnosis and treatment.

The term "DNA construct" in the present invention refers to a structural unit introduced into a host strain or cell via transformation, which enables the expression of a target protein. It includes not only the gene encoding the target protein but also essential regulatory elements, such as promoter sequences, that are operably linked to allow gene expression.

The "promoter" in the present invention refers to a nucleotide sequence located upstream of a gene that is operably linked within a host strain or cell. It is a specific DNA sequence of the DNA construct to which RNA polymerase binds to initiate transcription.

In the present invention, the 5'-untranslated region (5'-UTR) refers to the nontranslated portion of mRNA that is located adjacent to the coding region, which is translated into amino acids. Although once considered evolutionary "junk," it is now known to play a critical role in regulating gene expression.

In the present invention, the transcription factor binding site refers to a DNA region that functions to turn nearby specific genes on or off. This site may include at least one element selected from the group consisting of the promoter, enhancer, and silencer of the gene encoding the regulatory protein, although it is not limited thereto.

According to a specific embodiment of the present invention, the immune adjuvant is a DNA construct comprising at least one selected from the group consisting of gel-type adjuvants (metal salt compounds), oil-in-emulsion adjuvants (lipid-based particulates), cytokines, chemokines, particulate adjuvants, and microbial adjuvants. More specifically, the immune adjuvant is a DNA construct comprising at least one selected from the group consisting of IFN-α2, IL-2, IL-15, IL-21, IL-12, CXCR3, CCR5, T-cells, and B-cells. Even more specifically, the immune adjuvant is IL-15. Most specifically, the immune adjuvant is human IL-15, and is a gene or protein represented by Sequence No. 1.

According to another specific embodiment of the present invention, the flagellin is a DNA construct comprising at least one selected from the group consisting of flagellin A, B, C, D, and E. Specifically, the flagellin is flagellin A or B.

According to the present invention, the pBAD plasmid is used, and arabinose is used as an inducer.

The arabinose used in the present invention refers to a monosaccharide containing five carbon atoms, classified as an aldose with an aldehyde group, and has the chemical formula C₅H₁₀O₅. Due to the biosynthetic pathways, most sugars in nature primarily exist in the "D-form" or in a form structurally similar to D-glyceraldehyde.

In the present invention, the promoter of the gene encoding the regulatory protein may include any promoter that can be activated under various environmental conditions or developmental states of the host strain or cell. Preferably, it may be a weak promoter.

As used herein, the term "weak promoter" refers to a promoter that induces a transcription level of a transcript transcribed from a gene operably linked downstream to be 1 × 10⁻² or less, preferably 1 × 10⁻³ or less. Any promoter that achieves a transcript expression level of 1 × 10⁻³ or less may be included, for example, *E. coli* σ70 promoter; *E. coli* σS promoter; *E. coli* σ32 promoter; *B. subtilis* σA promoter; *B. subtilis* σB promoter; Salmonella-derived promoters such as K112706 or K112707; Bacteriophage T7 promoter; Bacteriophage SP6 promoter; Yeast-derived promoters; Promoters from eukaryotic cells. The term "DNA construct" in the present invention refers to a structural unit introduced into a host strain or cell via transformation, which enables the expression of a target protein. It includes not only the gene encoding the target protein but also essential regulatory elements, such as promoter sequences, that are operably linked to allow gene expression.

The "promoter" in the present invention refers to a nucleotide sequence located upstream of a gene that is operably linked within a host strain or cell. It is a specific DNA sequence of the DNA construct to which RNA polymerase binds to initiate transcription.

In the present invention, the 5'-untranslated region (5'-UTR) refers to the nontranslated portion of mRNA that is located adjacent to the coding region, which is translated into amino acids. Although once considered evolutionary "junk," it is now known to play a critical role in regulating gene expression.

In the present invention, the transcription factor binding site refers to a DNA region that functions to turn nearby specific genes on or off. This site may include at least one element selected from the group consisting of the promoter, enhancer, and silencer of the gene encoding the regulatory protein, although it is not limited thereto.

According to a specific embodiment of the present invention, the immune adjuvant is a DNA construct comprising at least one selected from the group consisting of gel-type adjuvants (metal salt compounds), oil-in-emulsion adjuvants (lipid-based particulates), cytokines, chemokines, particulate adjuvants, and microbial adjuvants. More specifically, the immune adjuvant is a DNA construct comprising at least one selected from the group consisting of IFN-α2, IL-2, IL-15, IL-21, IL-12, CXCR3, CCR5, T-cells, and B-cells. Even more specifically, the immune adjuvant is IL-15. Most specifically, the immune adjuvant is human IL-15, and is a gene or protein represented by Sequence No. 1.

According to another specific embodiment of the present invention, the flagellin is a DNA construct comprising at least one selected from the group consisting of flagellin A, B, C, D, and E. Specifically, the flagellin is flagellin A or B.

According to the present invention, the pBAD plasmid is used, and arabinose is used as an inducer.

The arabinose used in the present invention refers to a monosaccharide containing five carbon atoms, classified as an aldose with an aldehyde group, and has the chemical formula C₅H₁₀O₅. Due to the biosynthetic pathways, most sugars in nature primarily exist in the "D-form" or in a form structurally similar to D-glyceraldehyde.

In the present invention, the promoter of the gene encoding the regulatory protein may include any promoter that can be activated under various environmental conditions or developmental states of the host strain or cell. Preferably, it may be a weak promoter.

As used herein, the term "weak promoter" refers to a promoter that induces a transcription level of a transcript transcribed from a gene operably linked downstream to be 1 × 10⁻² or less, preferably 1 × 10⁻³ or less. Any promoter that achieves a transcript expression level of 1 × 10⁻³ or less may be included, for example, *E. coli* σ70 promoter; *E. coli* σS promoter; *E. coli* σ32 promoter; *B. subtilis* σA promoter; *B. subtilis* σB promoter; Salmonella-derived promoters K112706 or K112707; bacteriophage T7 promoter; bacteriophage SP6 promoter; yeast-derived promoters; eukaryotic cell-derived promoters such as I712004 or K076017; and plant-derived promoters, but is not limited thereto.

The *E. coli* σ70 promoter according to the present invention may be at least one selected from the group consisting of I14018, I14033, I14034, I732021, I742126, J01006, J23103, J23109, J23112, J23113, J23117, J23119, J23150, J23151, J44002, J48104, J56015, J64951, K088007, K119000, K119001, K1330002, K137029, K137030, K137031, K137032, K137085, K137086, K137087, K137088, K137089, K137090, K137091, K1585100, K1585101, K1585102, K1585103, K1585104, K1585105, K1585106, K1585110, K1585113, K1585115, K1585116, K1585117, K1585118, K1585119, K2486171, K256002, K256018, K256020, K256033, K292000, K823007, K823010, K823013, M13101, M13102, M13103, M13104, M13105, M13106, M13108, M13110, M31519, R1074, R1075, and S03331, but is not limited thereto.

The *E. coli* σS promoter according to the present invention may be J45992 or J45993, but is not limited thereto.

The *E. coli* σ32 promoter may be J45504, K1895002, or K1895003, but is not limited thereto.

The *B. subtilis* σA promoter may be at least one selected from the group consisting of K143012, K143013, K823000, K823002, and K823003, but is not limited thereto.

The *B. subtilis* σB promoter may be K143010, K143011, or K143013, but is not limited thereto.

The bacteriophage T7 promoter may be at least one selected from the group consisting of I719005, J34814, J64997, K113010, K113011, K113012, K1614000, R0085, R0180, R0181, R0182, R0183, Z0251, Z0252, and Z0253, but is not limited thereto.

The bacteriophage SP6 promoter may be J64998, but is not limited thereto.

The yeast-derived promoter may be at least one selected from the group consisting of I766557, J63005, K105027, K105028, K105029, K105030, K105031, K122000, K124000, K124002, K319005, M31201, K2365040, K2365036, K2365041, K2365042, K2365032, K2365051, K2365514, K2365515, and K2365516, but is not limited thereto.

The derived promoter may be at least one selected from the group consisting of I712004 or K076017, and the plant-derived promoter, but is not limited thereto.

The *E. coli* σ70 promoter according to the present invention may be at least one selected from the group consisting of I14018, I14033, I14034, I732021, I742126, J01006, J23103, J23109, J23112, J23113, J23117, J23119, J23150, J23151, J44002, J48104, J56015, J64951, K088007, K119000, K119001, K1330002, K137029, K137030, K137031, K137032, K137085, K137086, K137087, K137088, K137089, K137090, K137091, K1585100, K1585101, K1585102, K1585103, K1585104, K1585105, K1585106, K1585110, K1585113, K1585115, K1585116, K1585117, K1585118, K1585119, K2486171, K256002, K256018, K256020, K256033, K292000, K823007, K823010, K823013, M13101, M13102, M13103, M13104, M13105, M13106, M13108, M13110, M31519, R1074, R1075, and S03331, but is not limited thereto.

The *E. coli* σS promoter according to the present invention may be J45992 or J45993, but is not limited thereto.

The *E. coli* σ32 promoter may be J45504, K1895002, or K1895003, but is not limited thereto.

The *B. subtilis* σA promoter may be at least one selected from the group consisting of K143012, K143013, K823000, K823002, and K823003, but is not limited thereto.

The *B. subtilis* σB promoter may be K143010, K143011, or K143013, but is not limited thereto.

The bacteriophage T7 promoter may be at least one selected from the group consisting of I719005, J34814, J64997, K113010, K113011, K113012, K1614000, R0085, R0180, R0181, R0182, R0183, Z0251, Z0252, and Z0253, but is not limited thereto.

The bacteriophage SP6 promoter may be J64998, but is not limited thereto.

The yeast-derived promoter may be at least one selected from the group consisting of I766557, J63005, K105027, K105028, K105029, K105030, K105031, K122000, K124000, K124002, K319005, M31201, K2365040, K2365036, K2365041, K2365042, K2365032, K2365051, K2365514, K2365515, and K2365516, but is not limited thereto.

The plant-derived promoter of the present invention may be at least one selected from the group consisting of PLPR0203, PLPR0210, PLPR0177, PLPR0193, PLPR0507, PLPR0422, PLPR0228, PLPR0226, PLPR0223, PLPR0040, PLPR0465, PLPR0232, PLPR0205, PLPR0247, PLPR0328, PLPR0525, AtREG383, AtREG415, AtREG416, OsREG438, OsREG443, OsREG501, PpREG186, PpREG194, and PpREG197, but is not limited thereto.

For the purpose of the present invention, when a gene encoding the regulatory protein is operably linked downstream of the weak promoter, transcription of genes presents downstream of the first and/or second promoter can be specifically regulated to occur only upon administration of a substance that inhibits the regulatory protein, in contrast to when the gene is operably linked downstream of the first or second promoter.

According to another aspect of the present invention, the invention provides a recombinant vector comprising the DNA construct.

The recombinant vector of the present invention comprises the above-described DNA construct, wherein the regulatory protein is expressed under the control of a separate promoter, thereby allowing the genes operably linked downstream of the first and second promoters to be specifically and coordinately expressed only when an external substance inhibiting the regulatory protein is administered.

The recombinant vector of the present invention may be used as a means for expressing a protein in a cell and may be any known recombinant vector such as a plasmid vector, cosmid vector, or bacteriophage vector. The recombinant vector may be readily constructed by a person skilled in the art using known methods based on recombinant DNA technology.

In the present invention, specific examples of the recombinant vector include, but are not limited to, commercially available and widely used vectors such as pCDNA vectors, F, R1, RP1, Col, pBR322, ToL, Ti vectors, cosmids, and phages such as lambda, lambdoid, M13, Mu, pl, P22, Qµ, and T-even, T2, T3, T7, and plant viruses. The recombinant vector may be appropriately selected depending on the nature of the host cell for the intended purpose of the present invention.

In another embodiment of the present invention, a host cell or strain into which the recombinant vector comprising the DNA construct of the present invention is introduced is provided.

In the present invention, the host cells may include cells of mammalian, plant, insect, fungal, or cellular origin. Examples include bacterial cells such as *Escherichia coli, Streptomyces,* or *Salmonella* spp.; fungal cells such as yeast or *Pichia pastoris*; insect cells such as *Drosophila* or *Spodoptera* Sf9 cells; and animal cells such as CHO (Chinese hamster ovary), SP2/0 (mouse myeloma), human lymphoblastoid, COS, NSO (mouse myeloma), 293T, B16 melanoma, HT-1080, BHK (baby hamster kidney), HEK (human embryonic kidney), or PER.C6 (human retinal) cells, as well as plant cells. These host cells are not limited to these examples. For the purpose of this invention, the strain may be an anaerobic strain, for example selected from *Salmonella, Clostridium, Bifidobacterium,* and *Escherichia coli* species. Preferably, it may be selected from the group consisting of *Salmonella typhimurium, Salmonella choleraesuis,* and *Salmonella enteritidis,* and more preferably *Salmonella typhimurium,* though not limited thereto.

The strain in the present invention may be attenuated.

The term "attenuated" in this invention refers to the modification of microorganisms, such as through genetic engineering, to reduce toxicity or other adverse effects when administered to a patient. In cases where the strain is from the *Salmonella* genus, attenuation may be achieved by introducing mutations in at least one gene selected from the group consisting of *aroA, aroC, aroD, aroE, Rpur, htrA, ompR, ompF, ompC, galE, cya, crp, cyp, phoP, phoQ, rfaY, dksA, hupA, sipC, clpB, clpP, clpX, pab, nadA, pncB, pmi, rpsL, hemA, rfc, poxA, galU, cdt, pur, ssa, guaA, guaB, fliD, flgK, flgL, relA,* and *spoA,* but is not limited to these.

In the present invention, the method for modifying the above-described genes may be carried out by various gene deletion or disruption methods known in the art. For example, the deletion or disruption may be performed using methods such as homologous recombination, chemical mutagenesis, irradiation-induced mutagenesis, or transposon mutagenesis.

In this invention, the strain is designed to target the interior of tumor tissuesan environment characterized by incomplete vascularization and hypoxia, which is highly suitable for the proliferation of anaerobic strains. Therefore, when a recombinant vector capable of simultaneously and coordinately expressing both a real-time imageable reporter protein and an anticancer protein is introduced into such a strain, it can enable highly effective simultaneous diagnosis and treatment of cancer.

The recombinant vector of the present invention can be introduced into a host cell or strain through transformation (or transduction), and any transformation method known in the art can be employed, being readily performed using conventional techniques. Specifically, transformation methods commonly used for bacteria such as Salmonella strains, including CaCl₂ precipitation, the Hanahan method-which enhances transformation efficiency by using DMSO (dimethyl sulfoxide) with CaCl₂, electroporation, calcium phosphate precipitation, protoplast fusion, agitation with silicon carbide fibers, Agrobacterium-mediated transformation, PEG-mediated transformation, dextran sulfate, lipofectamine, and dry/inhibition-mediated transformation methods, may be used to introduce the recombinant vector into the strain; however, the invention is not limited thereto.

According to a specific embodiment of the present invention, the strain may be a cell selected from the group consisting of strains of *Salmonella, Clostridium, Bifidobacterium,* and *Escherichia coli.*

In another embodiment, the present invention provides a pharmaceutical composition for preventing or treating cancer, comprising the above strain as an active ingredient.

The term "prevention," as used herein, refers to the inhibition of the onset of a disease or disorder in a subject who has not been previously diagnosed with such a disease or disorder, but who is susceptible to developing it.

The term "treatment," as used herein, refers to any act that alleviates symptoms caused by cancer or benefits the subject by using the active ingredient of the present invention. It also includes attempts to achieve useful or desirable outcomes, including clinical results. A useful or desirable clinical outcome may include, but is not limited to: alleviation or improvement of one or more symptoms or conditions, reduction in the extent of the disease, stabilization of the disease state, inhibition of disease onset, suppression of disease spread, delay or postponement of disease progression or occurrence, improvement or mitigation of the disease condition, and partial or complete remission. In addition, "treatment" may also refer to an extension of patient survival beyond what would be expected in the absence of such treatment. Furthermore, "treatment" may include suppression or temporary delay of disease progression, and more preferably, is associated with the permanent cessation of disease progression. As understood by those skilled in the art, if treatment improves a specific disease state but results in adverse effects that outweigh all benefits attributed to the treatment in the patient, then the outcome may not be considered beneficial or desirable.

Accordingly, the composition of the present invention may serve as a therapeutic composition for these diseases on its own, or may be administered in combination with other anticancer agents as an adjuvant therapy for the aforementioned diseases. Therefore, as used in the present specification, the terms "treatment" or "therapeutic agent" are intended to encompass the meanings of "adjuvant treatment" or "adjuvant therapeutic agent."

As used herein, the term "pharmaceutical composition" may include, but is not limited to, formulations prepared in accordance with conventional methods, such as oral dosage forms including powders, granules, capsules, tablets, and aqueous suspensions; topical agents; suppositories; and sterile injectable solutions. The pharmaceutical composition of the present invention may include a pharmaceutically acceptable carrier. Such carriers may include, for oral administration: binders, lubricants, disintegrants, diluents, solubilizers, dispersing agents, stabilizers, suspending agents, colorants, flavoring agents, etc. For injectable formulations, they may include buffers, preservatives, analgesics, solubilizers, isotonic agents, stabilizers, and the like. For topical administration, the carriers may include bases, diluents, lubricants, and preservatives. The pharmaceutical composition of the present invention can be formulated in various forms by mixing with the pharmaceutically acceptable carriers described above. For example, oral formulations may be prepared in the form of tablets, lozenges, capsules, elixirs, suspensions, syrups, or wafers, and injectable formulations may be prepared as unit-dose ampoules or multi-dose forms. Other forms may include solutions, suspensions, tablets, capsules, and sustained-release formulations. Examples of suitable carriers, excipients, and diluents for formulation include, but are not limited to, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, or mineral oil. Additional components such as fillers, anti-caking agents, lubricants, wetting agents, flavoring agents, emulsifiers, and preservatives may also be included.

As used herein, the term "administration" or "to administer" refers to delivering a therapeutically effective amount of the composition of the present invention directly to a subject, such that the equivalent amount is formed within the subject's body. It includes introducing the composition into a patient by any appropriate method, and the route of administration may be via any conventional route capable of reaching the target tissue. Examples include, but are not limited to, oral administration, intraperitoneal administration, intravenous administration, intramuscular administration, subcutaneous administration, intradermal administration, intranasal administration, pulmonary administration, rectal administration, intracavitary administration, intraperitoneal administration, and intrathecal administration. The effective dose of the present invention may be adjusted according to various factors, including the type and severity of the disease, the nature and amount of active and other components in the composition, the type of formulation, the subject's age, weight, general health, sex, diet, time and route of administration, secretion rate of the composition, treatment duration, and concomitant medications. In adults, the therapeutic pharmaceutical composition may be administered in a volume of 50-500 ml per dose. In the case of compounds, it may be administered at a dose of 0.1 ng/kg to 10 mg/kg; for monoclonal antibodies, the same dosage range may apply. The frequency of administration can range from once to twelve times per day, and in the case of twelve administrations per day, it can be administered every two hours. Furthermore, the pharmaceutical composition of the present invention may be administered alone or in combination with other therapies known in the art for the treatment of cancer, such as chemotherapy, radiation, or surgery. It may also be administered in conjunction with other therapies designed to enhance immune responses, such as adjuvants or cytokines known in the art (or nucleic acids encoding such cytokines). Other standard delivery methods such as biolistic delivery or ex vivo treatment may also be used. In ex vivo treatment, for example, antigen-presenting cells (APCs), dendritic cells, peripheral blood mononuclear cells, or bone marrow cells may be harvested from a patient or suitable donor, activated with the pharmaceutical composition ex vivo, and then administered back to the patient.

As used herein, the term "therapeutically effective amount" refers to the amount of the pharmaceutical composition sufficient to provide a therapeutic or prophylactic effect in a subject to whom the composition is administered, and includes a "prophylactically effective amount."

According to a specific embodiment of the present invention, the cancer includes at least one selected from the group consisting of melanoma, fallopian tube cancer, brain cancer, small intestine cancer, esophageal cancer, lymphoma, gallbladder cancer, hematologic cancer, thyroid cancer, endocrine gland cancer, oral cancer, liver cancer, biliary tract cancer, colon cancer, rectal cancer, cervical cancer, ovarian cancer, kidney cancer, gastric cancer, duodenal cancer, prostate cancer, breast cancer, brain tumor, lung cancer, anaplastic thyroid cancer, uterine cancer, colorectal cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myeloid leukemia, and solitary myeloma.

According to a specific embodiment of the present invention, the cancer is one for which the pharmaceutical composition inhibits cancer growth or metastasis.

In the present invention, the term "cancer metastasis" refers to the property of cancer cells moving from the primary organ or site of origin to another location that is spatially separated. Metastatic cancer refers to cancer that has the potential to metastasize or has already metastasized. Such metastatic cancer may spread to the liver, lungs, bones, lymph nodes, or peritoneal cavity, although it is not limited thereto. Metastatic cancers are difficult to treat and tend to have more complex progression and treatment processes compared to the initial stages of therapy.

In the present invention, the term "cancer recurrence" refers to the reappearance of cancer after a period in which it was undetectable following treatment. Recurrent cancer refers to the cancer that results from such recurrence. In cases of recurrence, surgical resection is often difficult, and even if resection is possible, extensive surgery may be required. Furthermore, chemotherapy and radiotherapy may also be limited in such cases.

The pharmaceutical composition of the present invention may be characterized by being in the form of a capsule, tablet, granule, injection, ointment, powder, or beverage, and may be intended for use in humans.

The pharmaceutical composition of the present invention is not limited thereto, but may be formulated into oral dosage forms such as powders, granules, capsules, tablets, and aqueous suspensions, as well as topical agents, suppositories, and sterile injectable solutions using conventional methods. The pharmaceutical composition of the present invention may include pharmaceutically acceptable carriers. Pharmaceutically acceptable carriers may include, for oral administration, binders, glidants, disintegrants, diluents, solubilizers, dispersants, stabilizers, suspending agents, colorants, and flavoring agents. For injectable formulations, buffers, preservatives, analgesics, solubilizers, isotonic agents, and stabilizers may be used. For topical administration, bases, excipients, lubricants, and preservatives may be included. The pharmaceutical formulations of the present invention can be prepared in various forms by mixing with the above-described pharmaceutically acceptable carriers. For example, for oral administration, they may be formulated as tablets, troches, capsules, elixirs, suspensions, syrups, or wafers. In the case of injectables, they may be formulated in unit-dose ampoules or multi-dose vials. Additionally, they may be formulated as solutions, suspensions, tablets, capsules, or sustained-release preparations.

Examples of carriers, excipients, and diluents suitable for formulation include lactose, dextrose, sucrose, sorbitol, mannitol, xylitol, erythritol, maltitol, starch, acacia gum, alginates, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, or mineral oil. Furthermore, the composition may additionally include fillers, anti-caking agents, lubricants, wetting agents, flavoring agents, emulsifiers, and preservatives.

The administration routes of the pharmaceutical composition of the present invention are not limited to the following, but may include oral, intravenous, intramuscular, intra-arterial, intraosseous, intradural, intracardiac, transdermal, subcutaneous, intraperitoneal, intranasal, enteral, topical, sublingual, or rectal administration. Oral or parenteral administration is preferred.

As used in the present invention, "parenteral" refers to administration techniques including subcutaneous, intradermal, intravenous, intramuscular, intra-articular, intrabursal, intra-sternal, intradural, intralesional, and intracranial injections or infusions. The pharmaceutical composition of the present invention may also be administered in the form of a suppository for rectal administration.

The dosage of the pharmaceutical composition may vary depending on various factors including the activity of the specific compound used, age, body weight, general health, gender, diet, administration time, route of administration, excretion rate, drug combination, and severity of the disease to be prevented or treated. The dosage may be appropriately selected by those skilled in the art based on the patient's condition, weight, severity of the disease, formulation type, administration route, and duration. It may be administered at 0.0001 to 50 mg/kg or 0.001 to 50 mg/kg per day. Administration may be done once a day or in multiple divided doses. The dosage is not intended to limit the scope of the present invention in any way. The pharmaceutical composition according to the present invention may be formulated as pills, sugar-coated tablets, capsules, liquid formulations, gels, syrups, slurries, or suspensions.

According to another aspect of the present invention, the invention provides a strain transformed with recombinant vectors, each separately comprising a DNA construct encoding a flagellin protein and a DNA construct encoding an immune-enhancing protein.

In a specific embodiment of the present invention, the immune enhancer is selected from the group consisting of metal salt compounds (gel-type adjuvants), lipidic particle-type (oil-in-emulsion adjuvants), cytokines, chemokines, particulate adjuvants, and microbial adjuvants. Specifically, the immune enhancer is selected from the group consisting of IFN-α2, IL-2, IL-15, IL-21, IL-12, CXCR3, CCR5, T cells, and B cells. More specifically, the immune enhancer is IL-15. Still more specifically, the immune enhancer is human IL-15, which corresponds to the gene or protein represented by Sequence No. 1.

According to another aspect of the present invention, the invention provides a pharmaceutical composition for the prevention or treatment of cancer comprising the above-described strain as an active ingredient.

According to another aspect of the present invention, the invention provides a composition for the prevention or treatment of cancer comprising, as active ingredients, a flagellin protein or a nucleotide encoding the same, and an immune-enhancing protein or a nucleotide encoding the same.

In a specific embodiment of the present invention, the immune enhancer in the composition is selected from the group consisting of metal salt compounds (gel-type adjuvants), lipidic particle-type (oil-in-emulsion adjuvants), cytokines, chemokines, particulate adjuvants, and microbial adjuvants. Specifically, the immune enhancer is selected from the group consisting of IFN-α2, IL-2, IL-15, IL-21, IL-12, CXCR3, CCR5, T cells, and B cells. More specifically, the immune enhancer is IL-15. Still more specifically, the immune enhancer is human IL-15, represented by the gene or protein indicated as Sequence No. 1.

The gene of the present invention may be delivered via a gene delivery vector, but may also be administered in the form of a translated peptide to exert a similar pharmacological effect.

According to the present invention, the term "immune enhancer (adjuvant)" refers to an immune adjuvant used to increase antigenicity for vaccine development or to enhance non-specific immune responses to antigens, which may be used in cancer treatment and other applications. It enables various therapies by utilizing the host's inherent immune system and includes any substance capable of activating immune cells to induce the death of disease-related cells, such as cancer cells.

According to the present invention, the term "anti-cancer protein" refers to a peptide capable of directly or indirectly inducing the death of cancer cells. Examples include at least one selected from the group consisting of toxin proteins, antibodies specific to cancer antigens or fragments thereof, tumor suppressor proteins, angiogenesis inhibitors, cancer antigens, prodrug-converting enzymes, pro-apoptotic proteins, and flagellin, but are not limited thereto.

According to the present invention, the term "cytokine" refers to a protein secreted by immune cells, and the cytokines of the present invention include those that can be used in cancer immunotherapy to regulate host immune responses and induce the death of disease-related cells, such as cancer cells. Preferably, these may include IFN-α2, IL-2, IL-15, IL-21, and IL-12, but are not limited thereto.

According to the present invention, the term "chemokine" refers to a molecule that regulates cell migration between tissues and the localization and interaction of cells within tissues. It includes any substance capable of guiding leukocytes into the tumor microenvironment to mediate host responses to diseases such as cancer. Preferably, it may be CXCR3, CCR5, or the like, but is not limited thereto.

According to another aspect of the present invention, a pharmaceutical composition for the prevention or treatment of cancer is provided, comprising a recombinant strain and an immune checkpoint inhibitor as active ingredients.

In a specific embodiment of the present invention, the composition is characterized by being co-administered.

In a specific embodiment of the present invention, the immune checkpoint inhibitor includes at least one selected from the group consisting of Cytotoxic T-lymphocyte-associated antigen-4 (CTLA-4), Programmed cell death protein 1 (PD-1), Programmed death-ligand 1 (PD-L1), KIR, LAG3, CD137, OX40, CD47, CD276, CD27, and GITR.

In a more specific embodiment of the present invention, the immune checkpoint inhibitor is characterized in that it is Programmed death-ligand 1 (PD-L1).

In the present invention, the term "Programmed death-ligand 1 (PD-L1)" refers to a protein located on the surface of cancer cells or hematopoietic cells. It is also known as CD274 or B7-H1. When PD-L1 or PD-L2, proteins present on the surface of cancer cells, bind to PD-1, a protein on the surface of T cells, the T cells are prevented from attacking the cancer cells. Immune checkpoint inhibitors function by binding to the PD-1 receptor on T cells, thereby blocking this immune evasion mechanism of cancer cells.

In a specific embodiment of the present invention, the cancer includes at least one selected from the group consisting of melanoma, fallopian tube cancer, brain cancer, small intestine cancer, esophageal cancer, lymphoma, gallbladder cancer, blood cancer, thyroid cancer, endocrine gland cancer, oral cancer, liver cancer, bile duct cancer, colon cancer, rectal cancer, cervical cancer, ovarian cancer, kidney cancer, stomach cancer, duodenal cancer, prostate cancer, breast cancer, brain tumor, lung cancer, anaplastic thyroid cancer, uterine cancer, colon cancer, bladder cancer, ureteral cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary plasmacytoma.

In a specific embodiment of the present invention, the composition is a pharmaceutical composition for inhibiting cancer growth or metastasis.

According to another aspect of the present invention, a method for preventing or treating cancer is provided, comprising the step of administering a recombinant strain to a subject in need thereof. The recombinant strain comprises a gene encoding a flagellin protein and a gene encoding an adjuvant protein.

According to another aspect of the present invention, the use of a recombinant strain comprising a gene encoding a flagellin protein and a gene encoding an adjuvant protein is provided for the prevention or treatment of cancer.

### [Advantageous Effects]

The features and advantages of the present invention can be summarized as follows:
(a) The present invention provides a DNA construct comprising a gene encoding a flagellin protein; and
   a gene encoding an adjuvant protein.
(b) The present invention selectively kills only cancer cells for effective cancer treatment by designing an attenuated *Salmonella* strain to produce immunostimulatory substances within tumor tissues, thereby inducing a strong anti-tumor immune response. This approach significantly suppresses the size of both primary and metastatic tumors, and can be effectively utilized as a prophylactic or therapeutic composition that improves survival rates.

### [Description of Drawings]

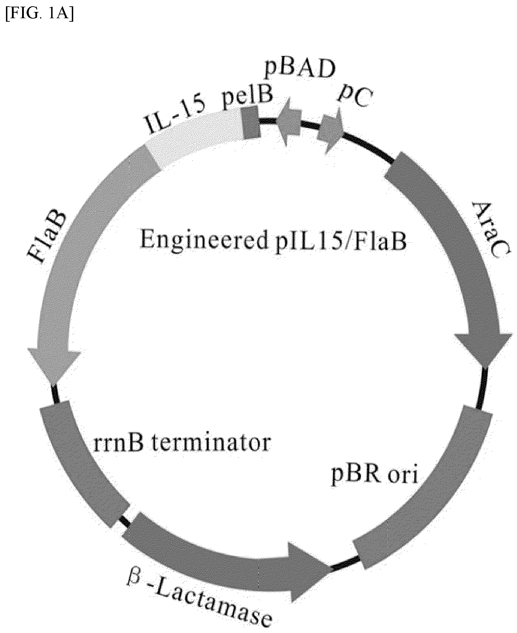

FIG. 1A illustrates a schematic diagram of the engineered plasmid pBAD IL15/FlaB according to one embodiment of the present invention.

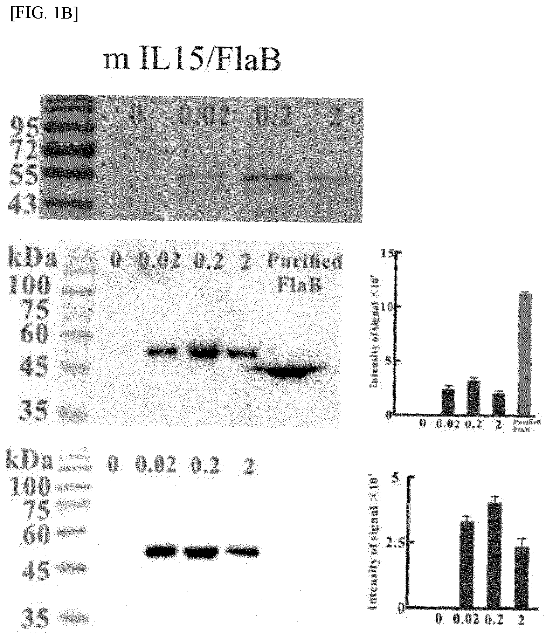

FIG. 1B shows the expression analysis of mouse IL15/FlaB by SDS-PAGE, anti-FlaB immunoblotting, and anti-mIL15 immunoblotting, according to one experimental example of the present invention.

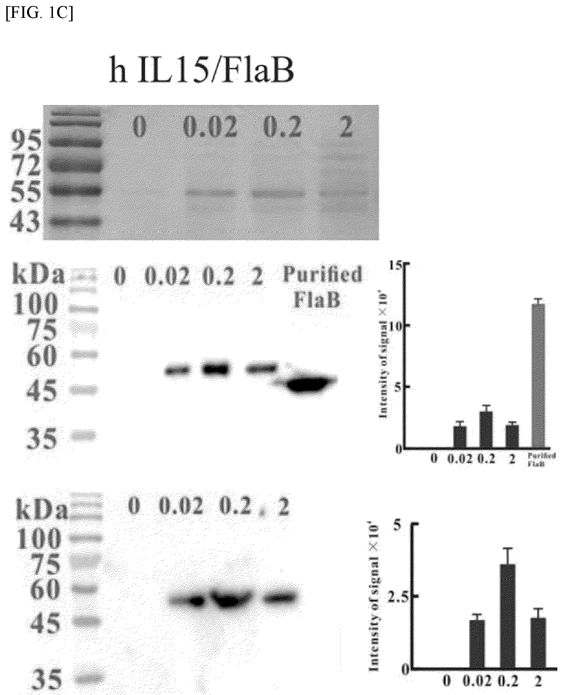

FIG. 1C shows the expression analysis of human IL15/FlaB by SDS-PAGE, anti-FlaB immunoblotting, and anti-hIL15 immunoblotting, according to one experimental example of the present invention.

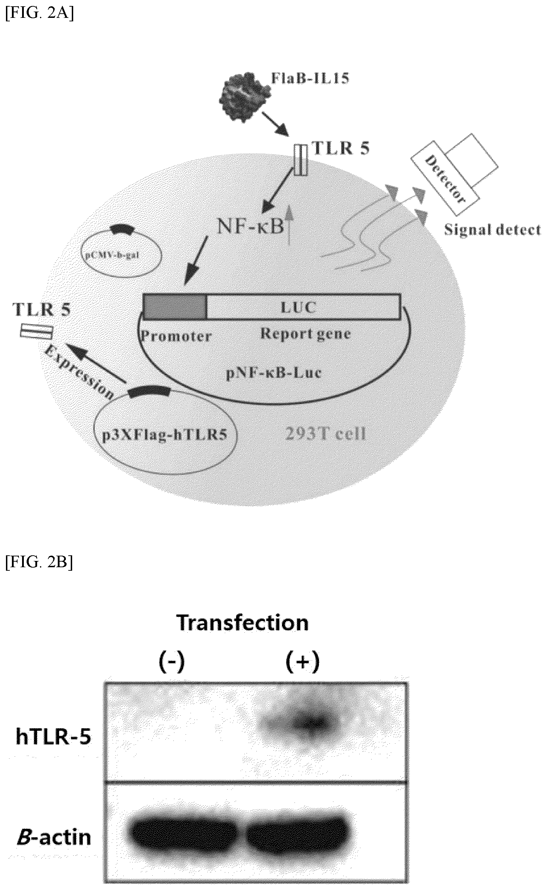

FIG. 2A presents a schematic of the IL15/FlaB activity detection system for analyzing the biological activity of IL15/FlaB secreted in vitro, according to one experimental example of the present invention.
FIG. 2B shows TLR5 expression following transfection with p3XFlag-hTLR5 (with simultaneous co-transfection of pCMV-b-gal and pNF-kB-luc), according to one experimental example of the present invention.

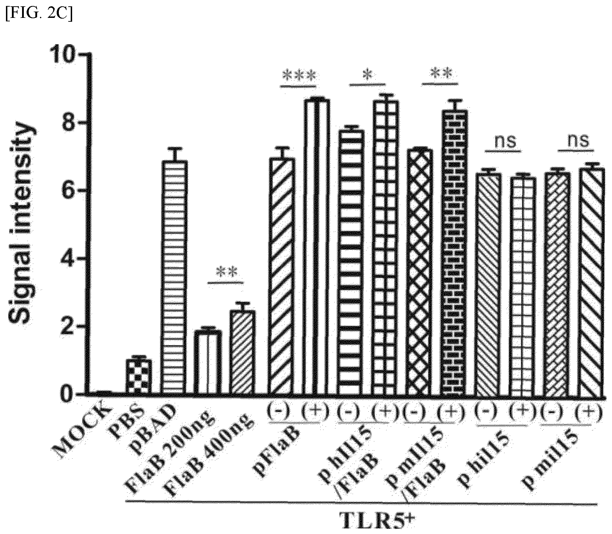

FIG. 2C illustrates that the signal intensity of IL15/FlaB interacts with TLR5-transfected cells and induces TLR5-mediated NF-kB transcription, according to one experimental example of the present invention.

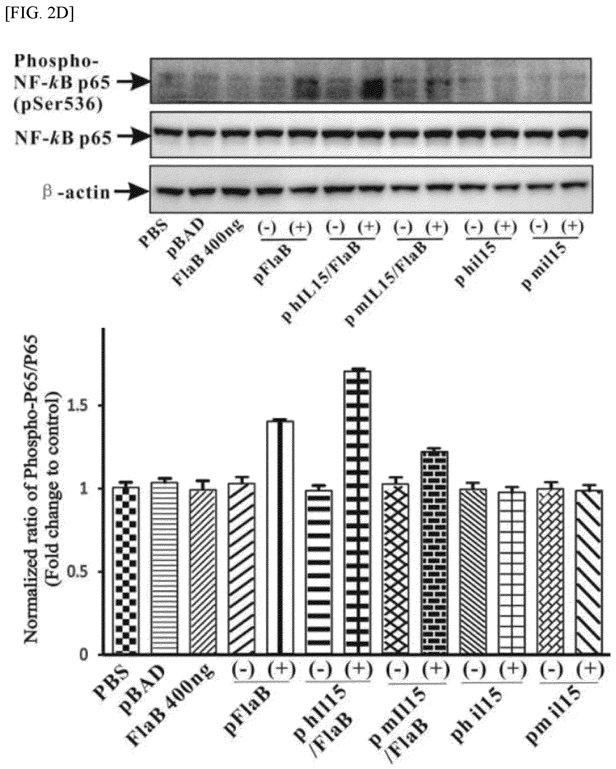

FIG. 2D shows the regulation of NF-kB and p-NF-kB signaling pathways following quantification of IL15/FlaB and immunoblot signals, according to one experimental example of the present invention.
FIG. 3 demonstrates the in vitro activity evaluation of IL15/FlaB, according to one experimental example of the present invention.
FIG. 4 shows the distribution of SL-Lux_pBAD-IL15/FlaB colonized in the tumor after injection of MC38 cells (1×10⁶ cells/mouse), according to one experimental example of the present invention.
FIG. 5 illustrates the anti-tumor effect of the recombinant strain after transplantation of MC38 tumor cells, according to one experimental example of the present invention.
FIG. 6 presents the results of tumor size changes and survival rate after re-implantation of tumors following MC38 tumor transplantation, according to one experimental example of the present invention.
FIG. 7 shows tumor size and survival rate after colonization of tumors following CT26 cell (1×10⁷ cfu/mouse) injection, according to one experimental example of the present invention.
FIG. 8 presents the results of tumor size and survival rate after re-implantation of tumors into Balb/c mice following CT26 tumor transplantation, according to one experimental example of the present invention.
FIG. 9 illustrates the tumor size in an untreated Balb/c group after re-implantation of tumors following CT26 tumor transplantation, according to one experimental example of the present invention.
FIG. 10 shows the tumor size in the SLpFlaB-treated Balb/c mouse group after re-implantation of tumors following CT26 tumor transplantation, according to one experimental example of the present invention.
FIG. 11 shows the tumor size in the SLphIL15/FlaB-treated Balb/c mouse group after re-implantation of tumors following CT26 tumor transplantation, according to one experimental example of the present invention.
FIG. 12 shows the tumor size in the SLpmIL15/FlaB-treated Balb/c mouse group after re-implantation of tumors following CT26 tumor transplantation, according to one experimental example of the present invention.
FIG. 13 presents the results before and after treatment in the 4T1 tumor model, according to one experimental example of the present invention.
FIG. 14 illustrates the effect of IL15/FlaB expression on 4T1 tumor metastasis, according to one experimental example of the present invention.
FIG. 15A demonstrates the anti-tumor efficacy of combination therapy using engineered bacteria and anti-PD-L1 antibodies in mice with metastatic malignant tumors, showing PD-L1 expression induced by 4T1-Luc and B16F10 cells. Cells were stained with anti-PD-L1 antibody and an isotype control before flow cytometry.
FIG. 15B shows a schematic diagram describing the immunotherapy schedule using engineered bacteria and anti-PD-L1 antibody, according to one embodiment of the present invention. The schematic indicates the timing of bacterial injection (SL), L-ara administration (+), and antibody injection. Isotype refers to the control antibody.
FIG. 15C presents bioluminescent light intensity (BLI) from ex vivo 4T1-Luc tumors and tumor weight on day 15, according to one embodiment of the present invention.
FIG. 15D shows the change in 4T1-Luc tumor size after treatment with engineered bacteria, according to one embodiment of the present invention.
FIG. 15E presents the Kaplan-Meier survival curve and survival rate of mice bearing 4T1 tumors, according to one embodiment of the present invention.
FIG. 15F shows the change in B16F10 tumor size after treatment with engineered bacteria, according to one embodiment of the present invention.
FIG. 15G presents the Kaplan-Meier survival curve and survival rate of mice bearing B16F10 tumors, according to one embodiment of the present invention.

### [Best Mode]

To evaluate the anti-cancer effect of the recombinant strains of the present invention, attenuated recombinant strains were intravenously administered at 1×10⁷ CFU into a CT26 mouse model. The tumor growth inhibitory effects of the recombinant strains-SLpBAD, SLphIL15, SLpmIL15, SLpFlaB, SLphIL15/FlaB, and SLpmIL15/FlaB-on CT26 cell-derived tumors were assessed.

As a result, administration of the SLphIL15/FlaB and SLpmIL15/FlaB recombinant strains significantly improved tumor suppression and mouse survival compared to the control group, confirming the anti-cancer efficacy of SLphIL15/FlaB and SLpmIL15/FlaB recombinant strains.

### [Mode for Invention]

Hereinafter, the present disclosure will be described in detail with reference to the following examples. However, the following examples are merely illustrative of the present disclosure, and the content of the present disclosure is not limited by the following examples.

### Example

### [Preparation Example 1] Cancer Cell Lines and Culture Conditions

The CT26 colon cancer cell lines CRL-2638 and HB-8064 (ATCC, USA), as well as the murine colorectal adenocarcinoma cell line MC38 (Massachusetts General Hospital and Harvard Medical School, USA; Chonnam National University, Korea), were used in the experiments.

The cells were cultured in high-glucose Dulbecco's Modified Eagle's Medium (DMEM; catalog number: #LM 001-05, Welgene, Korea) supplemented with 10% fetal bovine serum (FBS) and 1% penicillin-streptomycin, under conditions of 5% CO₂ at 37°C.

### [Preparation Example 2] Construction of Salmonella Strains Harboring Plasmids

The Salmonella strains used were *Salmonella typhimurium* (S. typhimurium) SLΔppGpp (*Δ*relA, *Δ*spoT) and CNC018 (*Δ*relA, *Δ*spoT, *Δ*SPI1, *Δ*SPI2), which are deficient in ppGpp.

Plasmids were introduced into the Salmonella strains via electroporation. Following transformation, each strain was cultured overnight in LB medium containing 100 µg/mL ampicillin. The overnight cultures were then diluted 1:100 into fresh LB medium containing ampicillin and cultured in a shaking incubator at 200 rpm and 37°C. After incubation, the cultures were centrifuged to collect bacterial pellets, which were washed with PBS buffer and used in subsequent experiments.

### [Preparation Example 3] Experimental Animal Model Preparation

C57BL/6 and BALB/c mice (Orient Co., Korea) aged 5 to 6 weeks and weighing 20 to 30 g were used. The tumor animal models were established by subcutaneously injecting MC38 or CT26 cells from Preparation Example 1 into the flanks of the mice.

For imaging and tumor size evaluation of the tumor models, 2% isoflurane was used for anesthesia. During surgical procedures, ketamine (200 mg/kg) and xylazine (10 mg/kg) were administered.

Tumor volume (mm³) was calculated using the formula: (length × height × width)/2. Mice were euthanized when the tumor volume exceeded 1500 mm³.

### [Experimental Example 1] Evaluation of Recombinant Strain Protein Expression and Activity

### - Comparison Between Recombinant and Existing Strains

The recombinant strains SLphIL15/FlaB and SLpmIL15/FlaB, and the control strain SLpEmpty, were cultured overnight in LB liquid medium containing ampicillin. The cultures were then diluted 1:100 into fresh LB medium and further cultured until the OD600 value reached 0.5 to 0.7. At that point, L-arabinose was added to the culture to a final concentration of 0.2%, followed by incubation in a shaking incubator at 200 rpm and 37°C. Growth patterns of the strains were analyzed by measuring OD600 values over time.

### [Experimental Example 2] Verification of Immunological Effects of Recombinant Strains

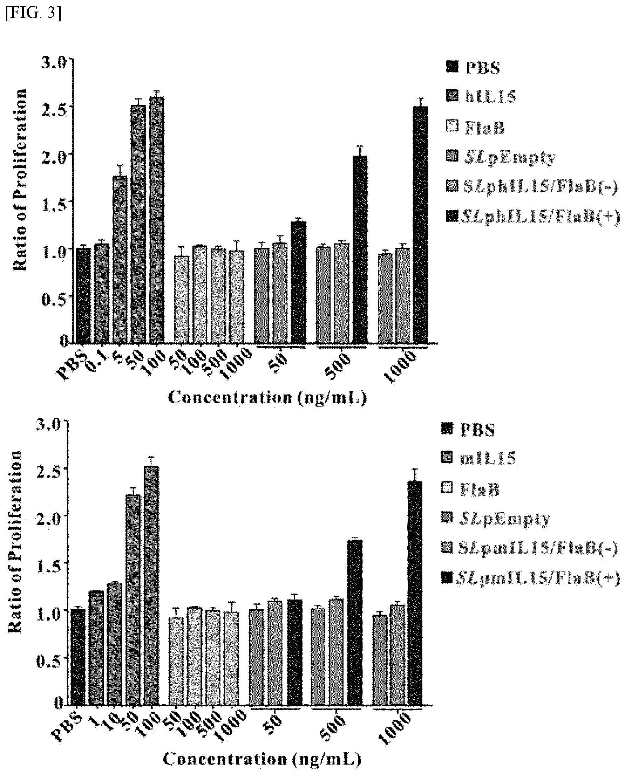

To evaluate the immunological effects of the recombinant strains, an *in vitro* cytotoxicity assay was performed using the CTLL-2 cell line. The CTLL-2 cells were treated with the recombinant strains SLphIL15/FlaB and SLpmIL15/FlaB, as well as with the control strains hIL15, mIL15, and SLpEmpty. The proliferation rate of the CTLL-2 cells was then analyzed and presented in FIG. 3.

As shown in FIG. 3, unlike the SLpEmpty strain, which showed no change in CTLL-2 proliferation, the recombinant strain SLpmIL15/FlaB expressing IL15/FlaB significantly enhanced CTLL-2 cell proliferation, thereby confirming the immune-enhancing effect of the SLpmIL15/FlaB recombinant strain. The sequences of human and mouse IL-15 are shown in Table 1.

**[Table 1]**

| **Designation of Sequence** | **Sequence** |
|---|---|
| **Human IL-15** | ATGAATTGGGTCAACGTGATTAGCGATCTGAAAAAGATCGAAG ATCTCATTCAGAGCATGCATATTGATGCTACGCTGTATACAGA ATCCGACGTGCATCCGAGCTGCAAAGTTACTGCAATGAAGTGC TTCCTGCTGGAACTGCAGGTAATAAGCCTGGAGAGCGGTGACG CGAGCATCCATGATACAGTTGAAAATCTGATAATCCTGGCAAA CAACTCGCTGAGCAGCAATGGTAATGTGACGGAATCAGGCTGC AAAGAATGTGAGGAGCTGGAAGAAAAAAACATCAAAGAGTTC CTGCAGAGCTTCGTCCACATTGTTCAAATGTTTATTAACACCAG CTAA |
| **Mouse IL-15** | ATGAATTGGATCGATGTACGCTATGACCTGGAAAAAATTGAAT CACTGATTCAGAGCATTCATATCGACACTACCCTGTATACCGA TAGCGATTTTCATCCGTCCTGCAAAGTGACAGCAATGAACTGT TTCCTGCTCGAACTGCAGGTCATTCTGCACGAGTATAGCAATA TGACCCTGAACGAGACGGTGCGTAATGTTCTGTACCTGGCGAA TAGCACCCTGAGCAGCAATAAAAACGTTGCTGAAAGCGGATGT AAAGAATGCGAGGAACTCGAGGAAAAGACGTTTACTGAATTT CTGCAGAGCTTCATTCGGATAGTCCAAATGTTCATTAACACGT CA |

### [Experimental Example 3] Evaluation of Tumor-Targeting Effect of Recombinant Strains

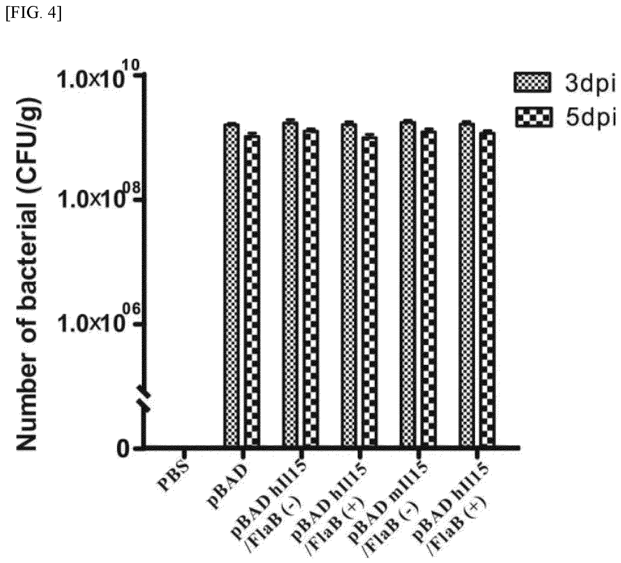

To evaluate the tumor-targeting effect of the recombinant strains, *in vivo* experiments were conducted to assess the number of recombinant bacteria within tumors and visualize their targeting via imaging. In the MC38 mouse model, 1×10⁷ CFU of the attenuated SLΔppGpp-Lux recombinant strain was administered intravenously. The number of SLΔppGpp-Lux bacteria in the tumor was then quantified, and fluorescence images expressed by the strain within the tumor were obtained and presented in FIG. 4.

As shown in FIG. 4, the recombinant bacteria were found to be specifically present within the tumor, confirming that the recombinant strain selectively targets tumor cells.

### [Experimental Example 4] Evaluation of Antitumor Effect of Recombinant Strains (1)

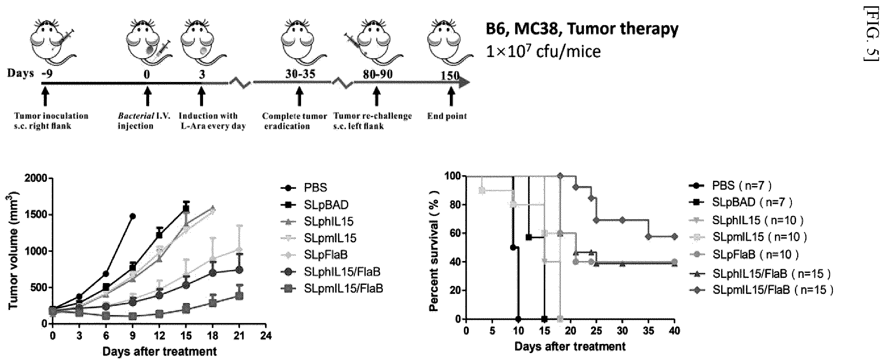

To evaluate the antitumor effect of the recombinant strains, *in vivo* experiments were performed following the method described in FIG. 5, assessing the growth-inhibitory effect of SLpBAD, SLphIL15, SLpmIL15, SLpFlaB, SLphIL15/FlaB, and SLpmIL15/FlaB recombinant strains on the MC38 cell line. In the MC38 mouse model, each attenuated recombinant strain was administered intravenously at 1×10⁷ CFU, and the results regarding tumor volume and mouse survival rate are shown in FIG. 5.

As depicted in FIG. 5, administration of SLphIL15/FlaB and SLpmIL15/FlaB recombinant strains led to a significant increase in tumor suppression and mouse survival rate compared to the control group, thereby confirming the antitumor effects of SLphIL15/FlaB and SLpmIL15/FlaB recombinant strains.

### [Experimental Example 5] Evaluation of Tumor Recurrence Suppression by Recombinant Strains

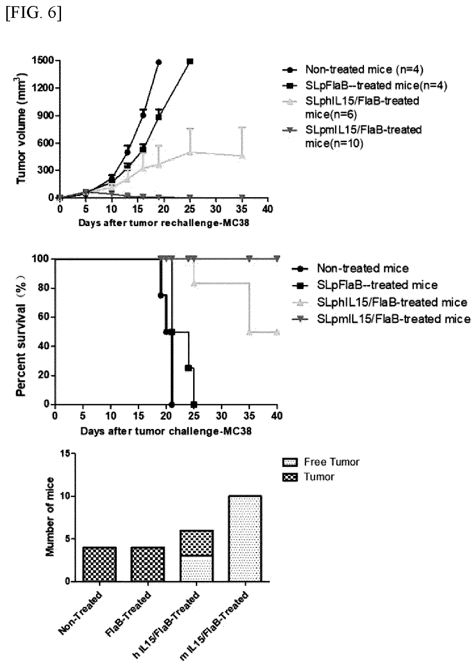

To evaluate the effect of recombinant strains in suppressing tumor recurrence, *in vivo* experiments were conducted to assess the tumor-suppressive effects of SLphIL15/FlaB and SLpmIL15/FlaB recombinant strains on the MC38 cell line. In the MC38 mouse model, each recombinant strain was intravenously administered at a dose of 1×10⁷ CFU. After complete tumor regression, MC38 cells were re-inoculated 90 days later. The size of the recurrent tumors and the survival rate of the mice were evaluated and are shown in FIG. 6.

As shown in FIG. 6, mice treated with SLphIL15/FlaB and SLpmIL15/FlaB recombinant strains showed significantly suppressed tumor regrowth and markedly improved survival rates compared to the control group, confirming the tumor recurrencesuppressing effects of the SLphIL15/FlaB and SLpmIL15/FlaB recombinant strains.

### [Experimental Example 6] Evaluation of Antitumor Effect of Recombinant Strains (2)

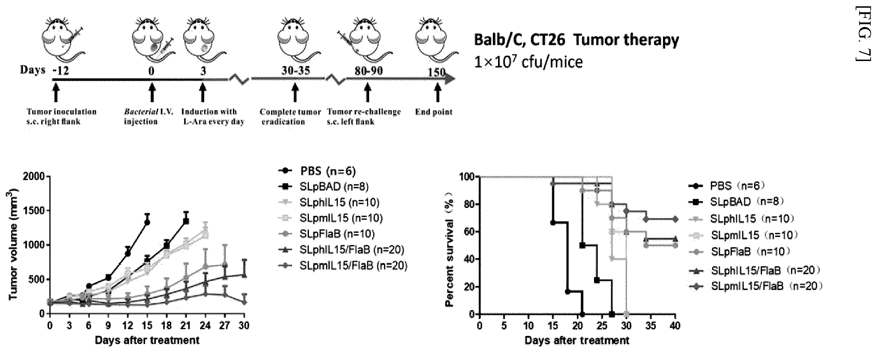

To evaluate the antitumor effect of the recombinant strains, *in vivo* experiments were conducted as shown in FIG. 7 to examine the growth-inhibitory effects of SLpBAD, SLphIL15, SLpmIL15, SLpFlaB, SLphIL15/FlaB, and SLpmIL15/FlaB recombinant strains on the CT26 cell line. In the CT26 mouse model, each attenuated recombinant strain was intravenously administered at a dose of 1 × 10⁷ CFU. Tumor volumes and mouse survival rates were evaluated and are presented in FIG. 7.

As shown in FIG. 7, administration of SLphIL15/FlaB and SLpmIL15/FlaB recombinant strains led to significantly enhanced tumor suppression and improved mouse survival rates compared to the control group, confirming the antitumor efficacy of SLphIL15/FlaB and SLpmIL15/FlaB recombinant strains.

### [Experimental Example 7] Evaluation of Tumor Recurrence Suppression by Recombinant Strains

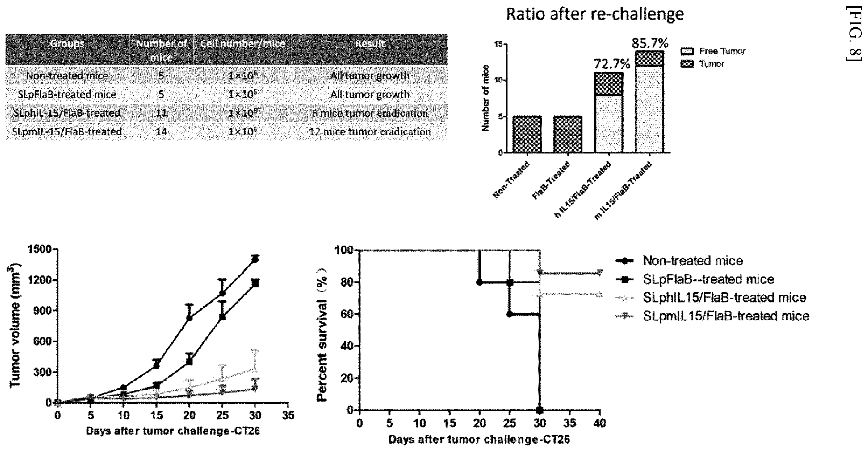

To evaluate the tumor recurrence suppression effect of recombinant strains, *in vivo* experiments were conducted to assess the tumor-inhibitory effects of SLphIL15/FlaB and SLpmIL15/FlaB recombinant strains on the CT26 cell line. In the CT26 mouse model, each attenuated recombinant strain was intravenously administered at a dose of 1×10⁷ CFU. After complete tumor regression, CT26 cells were re-inoculated 90 days later, and the tumor size and mouse survival rate upon rechallenge were evaluated and are shown in FIG. 8.

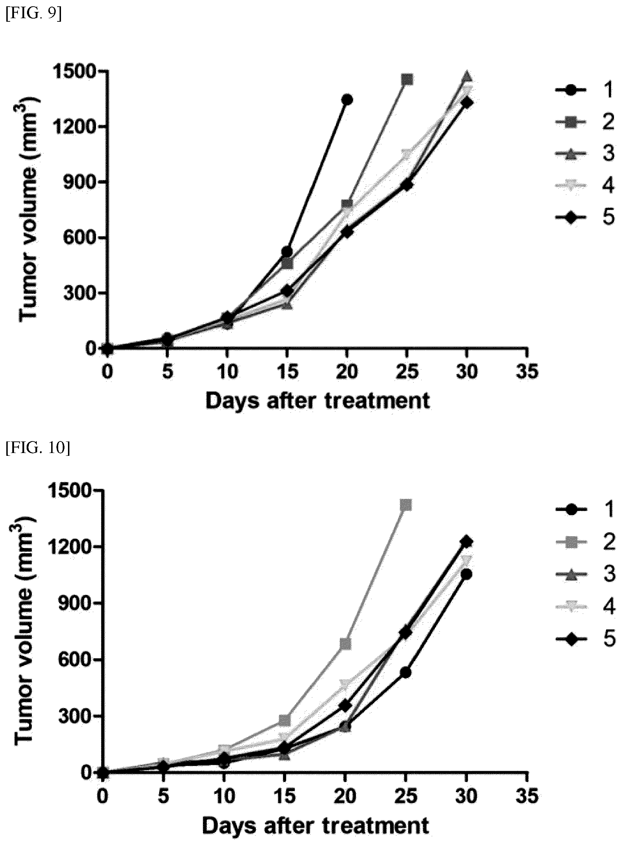

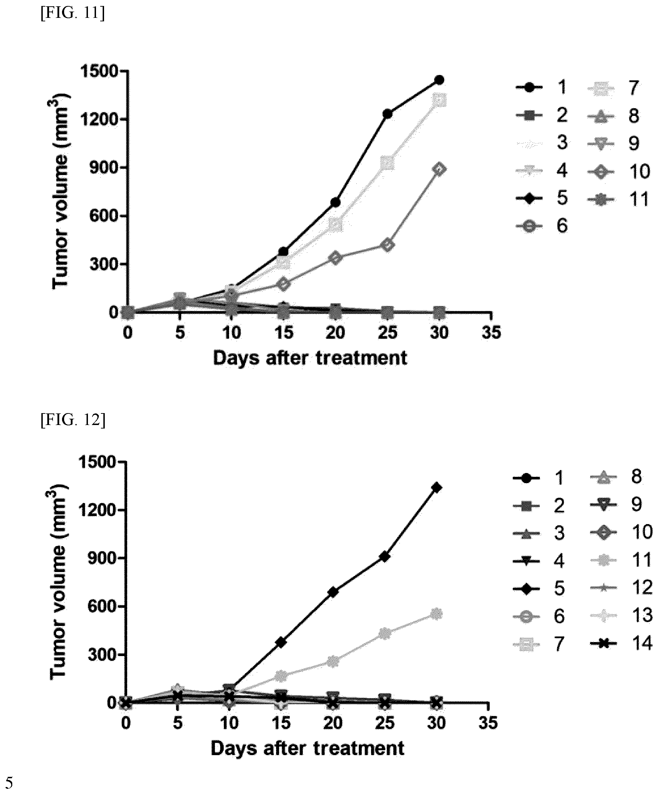

As shown in FIG. 8, mice treated with SLphIL15/FlaB and SLpmIL15/FlaB recombinant strains exhibited significantly enhanced suppression of tumor regrowth and improved survival compared to the control group, confirming the tumor recurrenceinhibitory effects of the SLphIL15/FlaB and SLpmIL15/FlaB recombinant strains. In the non-treated group and the group treated with SLpFlaB, all mice showed tumor regrowth. In contrast, the SLphIL15/FlaB group showed tumor eradication in 8 out of 11 mice (72.7%), while the SLpmIL15/FlaB group showed tumor eradication in 12 out of 14 mice (85.7%) (FIG. 9 to FIG. 12).

### [Experimental Example 8] Evaluation of Antimetastatic Effect of Recombinant Strains

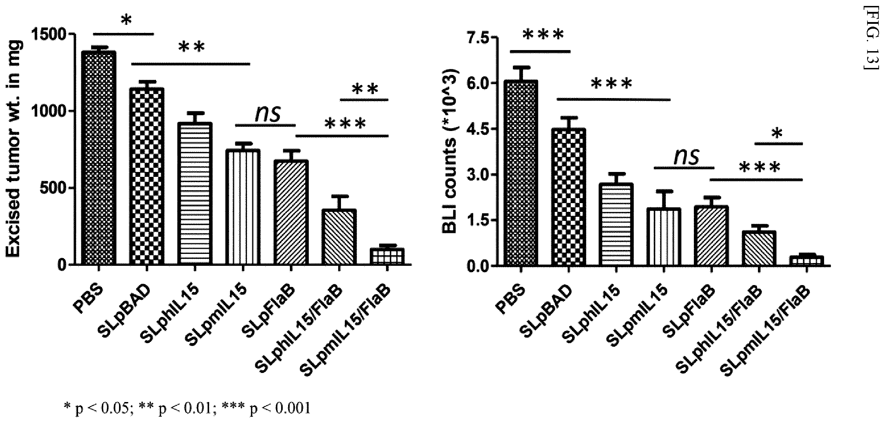

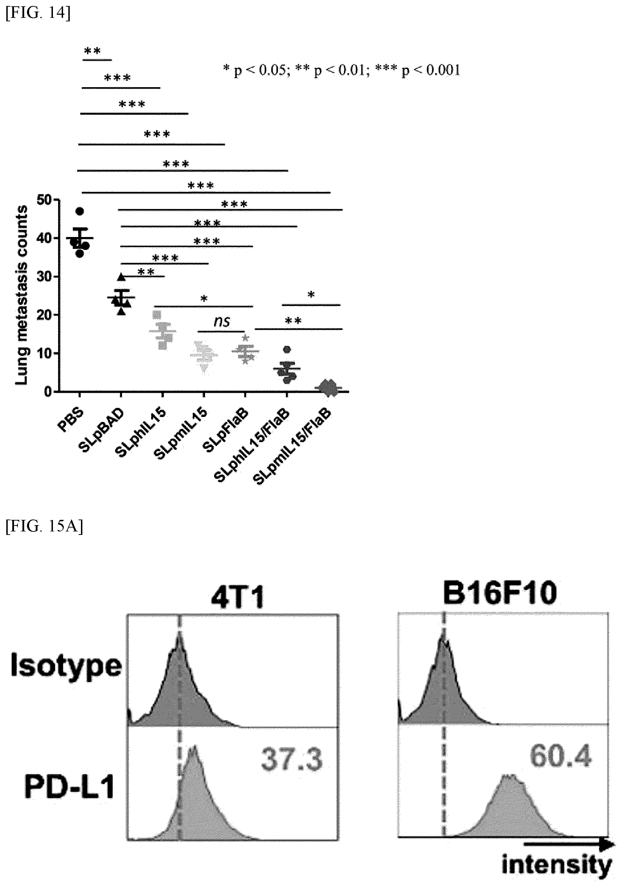

To evaluate the antimetastatic effect of recombinant strains, *in vivo* experiments were conducted to assess the tumor-inhibitory effects of SLpBAD, SLphIL15, SLpmIL15, SLpFlaB, SLphIL15/FlaB, and SLpmIL15/FlaB recombinant strains on the 4T1-Luc cell line. Mice were implanted with luciferase-expressing 4T1-Luc cells to establish a tumor model. Subsequently, each attenuated recombinant strain was intravenously administered at a dose of 1×10⁷ CFU. Tumor localization was imaged and is shown in FIG. 13, and the number of tumor nodules metastasized to the lungs was quantified and presented in FIG. 14.

As shown in FIG. 13 and FIG. 14, mice treated with SLphIL15/FlaB and SLpmIL15/FlaB recombinant strains showed no tumor metastasis to the lungs, indicating that SLphIL15/FlaB and SLpmIL15/FlaB strains have a pronounced inhibitory effect on tumor metastasis.

### [Experimental Example 9] Evaluation of Antitumor Effect of Combination Therapy with Recombinant Strains and Immune Checkpoint Inhibitor

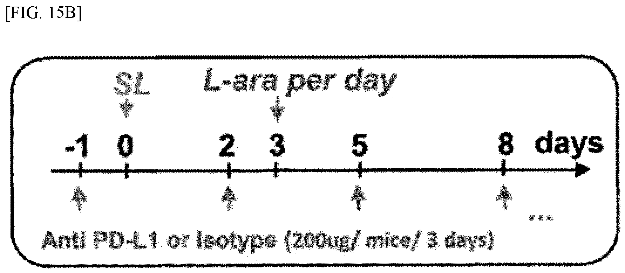

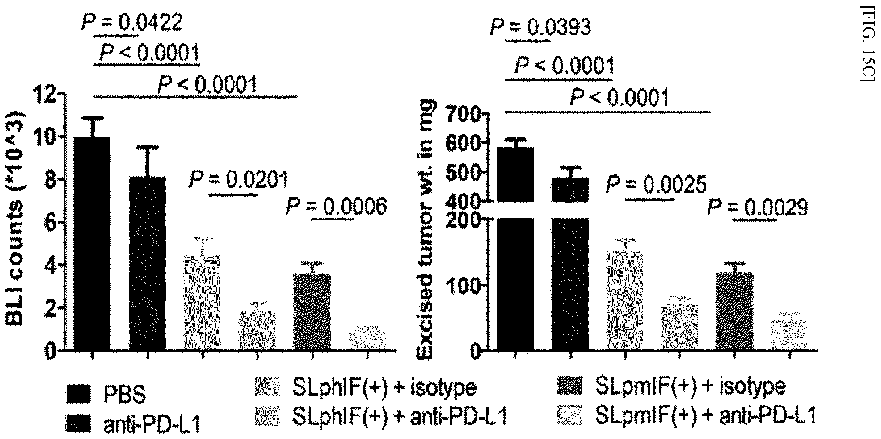

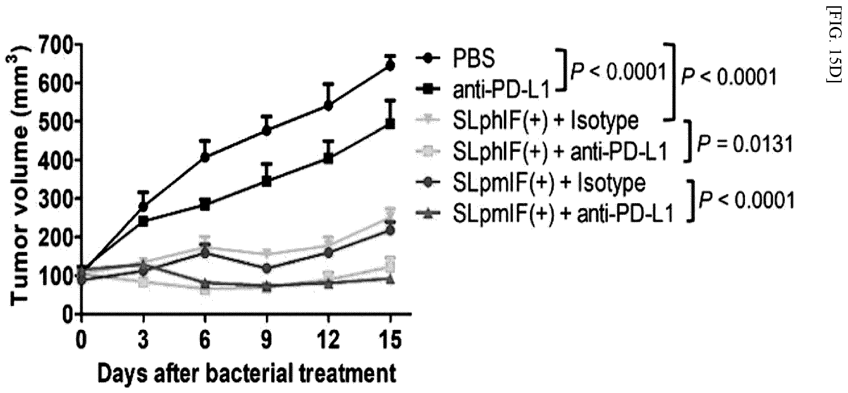

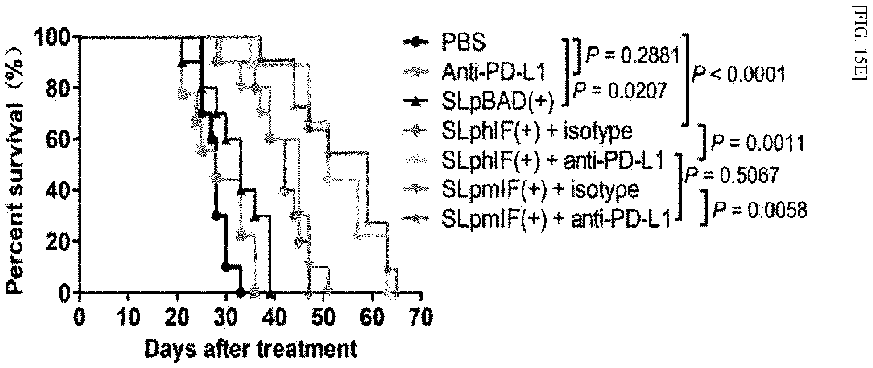

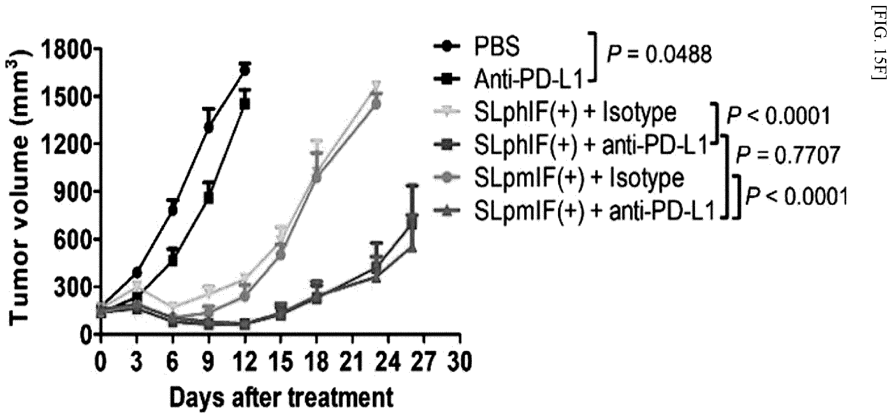

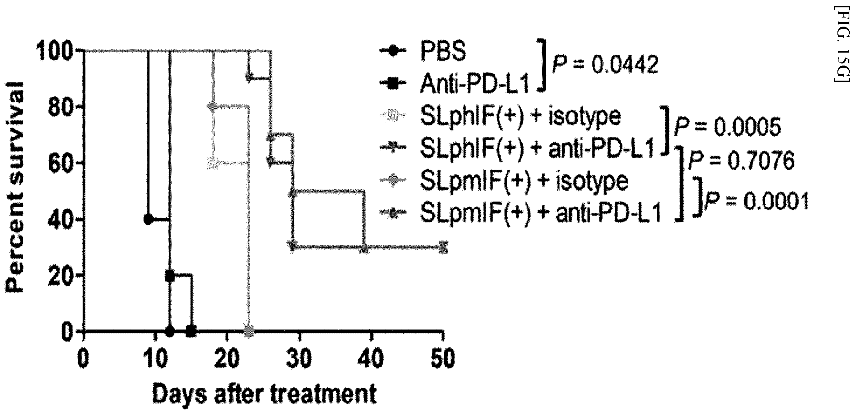

To evaluate the antitumor effect of combination therapy with recombinant strains and the immune checkpoint inhibitor anti-PD-L1, *in vivo* experiments were conducted in 4T1-Luc and B 16F10 tumor models. Mice were treated with PBS, anti-PD-L1, SLphIF(+) + isotype, SLphIF(+) + anti-PD-L1, SLpmIF(+) + isotype, or SLpmIF(+) + anti-PD-L1, and tumor size and mouse survival rates were assessed. As a result, mice treated with SLphIF(+) and SLpmIF(+) strains showed significantly greater tumor suppression compared to the PBS control. Notably, co-administration of SLphIF(+) + anti-PD-L1 and SLpmIF(+) + anti-PD-L1 demonstrated significantly enhanced tumor inhibition compared to treatment with recombinant strains alone. In the 4T1-Luc tumor model, combination treatment also led to a marked improvement in mouse survival (FIG. 15D and FIG. 15E). Furthermore, a substantial increase in survival was also observed in mice bearing B16F10 tumors treated with the combination therapy (FIG. 15F and FIG. 15G).

While specific aspects of the present invention have been described in detail above, it is clear to those skilled in the art that such detailed descriptions merely illustrate preferred embodiments and do not limit the scope of the invention. Therefore, the true scope of the present invention is defined by the appended claims and their equivalents.

### [Industrial Applicability]

The present invention was designed to selectively kill only cancer cells for the development of an effective cancer prevention or treatment agent. By engineering an attenuated *Salmonella* strain to produce immune-stimulating substances within tumor tissues, it induces a strong anti-tumor immune response, demonstrating effective treatment not only for primary tumors but also for metastatic cancers. As the pharmaceutical composition of the present invention specifically targets and kills cancer cells within the body, thereby improving survival rates, it is expected to have broad applications in the field of cancer therapy.

### [Sequence Lists Free Text]

**SEQ ID NOs: 1. Human IL-15** 
ATGAATTGGGTCAACGTGATTAGCGATCTGAAAAAGATCGAAGAT
CTCATTCAGAGCATGCATATTGATGCTACGCTGTATACAGAATCCGACGTG
CATCCGAGCTGCAAAGTTACTGCAATGAAGTGCTTCCTGCTGGAACTGCAG
GTAATAAGCCTGGAGAGCGGTGACGCGAGCATCCATGATACAGTTGAAAA
TCTGATAATCCTGGCAAACAACTCGCTGAGCAGCAATGGTAATGTGACGG
AATCAGGCTGCAAAGAATGTGAGGAGCTGGAAGAAAAAAACATCAAAGA
GTTCCTGCAGAGCTTCGTCCACATTGTTCAAATGTTTATTAACACCAGCTAA
**SEQ ID NOs: 2. Mouse IL-15** 
ATGAATTGGATCGATGTACGCTATGACCTGGAAAAAATTGAATCAC
TGATTCAGAGCATTCATATCGACACTACCCTGTATACCGATAGCGATTTTC
ATCCGTCCTGCAAAGTGACAGCAATGAACTGTTTCCTGCTCGAACTGCAGG
TCATTCTGCACGAGTATAGCAATATGACCCTGAACGAGACGGTGCGTAATG
TTCTGTACCTGGCGAATAGCACCCTGAGCAGCAATAAAAACGTTGCTGAAA
GCGGATGTAAAGAATGCGAGGAACTCGAGGAAAAGACGTTTACTGAATTT
CTGCAGAGCTTCATTCGGATAGTCCAAATGTTCATTAACACGTCA
**SEQ ID NOs: 3. PelB (pectate lyase B)**

ATGAAATACCTATTGCCTACGGCAGCCGCTGGATTGTTATTACTCG
CGGCCCAACCGGCCATGGCC

## Claims

1. A DNA construct comprising:
a gene encoding a flagellin protein; and
a gene encoding an adjuvant protein.

2. The DNA construct of claim 1,
wherein the adjuvant is selected from the group consisting of gel-type adjuvants, oil-in-emulsion adjuvants, cytokines, chemokines, particulate adjuvants, and microbial adjuvants.

3. The DNA construct of claim 1,
wherein the flagellin is selected from the group consisting of flagellin A, flagellin B, flagellin C, flagellin D, and flagellin E.

4. A recombinant vector comprising the DNA construct according to any one of claims 1 to 3.

5. A cell into which the recombinant vector of claim 4 is introduced.

6. The cell of claim 5,
wherein the strain is at least one selected from the group consisting of *Salmonella, Clostridium, Bifidobacterium, Listeria, Enterococcus, Yersinia,* and *Escherichia coli.*

7. A pharmaceutical composition for the prevention or treatment of cancer, comprising the strain of claim 5 as an active ingredient.

8. The pharmaceutical composition of claim 7,
wherein the cancer is at least one selected from the group consisting of melanoma, fallopian tube cancer, brain cancer, small intestine cancer, esophageal cancer, lymphoma, gallbladder cancer, blood cancer, thyroid cancer, endocrine gland cancer, oral cancer, liver cancer, biliary tract cancer, colon cancer, rectal cancer, cervical cancer, ovarian cancer, kidney cancer, gastric cancer, duodenal cancer, prostate cancer, breast cancer, brain tumor, lung cancer, anaplastic thyroid carcinoma, uterine cancer, colorectal cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myeloid leukemia, and solitary plasmacytoma.

9. The pharmaceutical composition of claim 7,
wherein the cancer treatment is for inhibiting cancer growth or metastasis.

10. A strain transformed with recombinant vectors, each separately comprising a DNA construct encoding a flagellin protein and a DNA construct encoding an immune-enhancing protein.

11. The strain of claim 10,
wherein the adjuvant is selected from the group consisting of gel-type adjuvants, oil-in-emulsion adjuvants, cytokines, chemokines, particulate adjuvants, and microbial adjuvants.

12. A pharmaceutical composition for the prevention or treatment of cancer, comprising the strain of claim 5 or claim 10 as an active ingredient.

13. A composition for the prevention or treatment of cancer,
comprising, as active ingredients:
a flagellin protein or a nucleotide encoding the same; and
an adjuvant protein or a nucleotide encoding the same.

14. The composition of claim 13,
wherein the adjuvant is selected from the group consisting of gel-type adjuvants, oil-in-emulsion adjuvants, cytokines, chemokines, particulate adjuvants, and microbial adjuvants.

15. A pharmaceutical composition for the prevention or treatment of cancer,
comprising the strain of claim 5 and an immune checkpoint inhibitor as active ingredients.

16. The pharmaceutical composition of claim 15,
wherein the composition is administered in combination.

17. The pharmaceutical composition of claim 15,
wherein the immune checkpoint inhibitor comprises at least one selected from the group consisting of cytotoxic T-lymphocyte-associated antigen-4 (CTLA-4), programmed cell death protein 1 (PD-1), programmed death-ligand 1 (PD-L1), KIR, LAG3, CD137, OX40, CD47, CD276, CD27, and GITR.

18. The pharmaceutical composition of claim 15,
wherein the cancer is at least one selected from the group consisting of melanoma, fallopian tube cancer, brain cancer, small intestine cancer, esophageal cancer, lymphoma, gallbladder cancer, blood cancer, thyroid cancer, endocrine gland cancer, oral cancer, liver cancer, biliary tract cancer, colon cancer, rectal cancer, cervical cancer, ovarian cancer, kidney cancer, gastric cancer, duodenal cancer, prostate cancer, breast cancer, brain tumor, lung cancer, anaplastic thyroid carcinoma, uterine cancer, colorectal cancer, bladder cancer, ureter cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myeloid leukemia, chronic lymphocytic leukemia, chronic myeloid leukemia, and solitary plasmacytoma.

19. The pharmaceutical composition of claim 15,
wherein the composition inhibits cancer growth or metastasis.

20. A method for preventing or treating cancer, comprising administering a recombinant strain to a subject in need thereof,
wherein the recombinant strain comprises:
a gene encoding a flagellin protein; and
a gene encoding an adjuvant protein.

21. Use of a recombinant strain comprising:
a gene encoding a flagellin protein; and
a gene encoding an adjuvant protein,
for the prevention or treatment of cancer.
